(19) Europäisches Patentamt
European Patent Office
Office européen des brevets

(11) Publication number: **0 060 617**
**A1**

(12) # EUROPEAN PATENT APPLICATION

(21) Application number: **82300641.6**

(22) Date of filing: **09.02.82**

(51) Int. Cl.³: **C 07 C 69/74**, C 07 C 147/06, C 07 C 149/36, C 07 C 103/38, C 07 C 93/26, C 07 C 149/18, C 07 C 93/227, A 01 N 53/00, C 07 C 67/14

(30) Priority: **18.03.81 GB 8108410**

(43) Date of publication of application: **22.09.82**
**Bulletin 82/38**

(84) Designated Contracting States: **CH DE FR GB IT LI NL**

(71) Applicant: **IMPERIAL CHEMICAL INDUSTRIES PLC, Imperial Chemical House Millbank, London SW1P 3JF (GB)**

(72) Inventor: **Crowley, Patric Jelf, 56 Ellis Road, Crowthorne Berkshire (GB)**

(74) Representative: **Bishop, Nigel Douglas et al, Imperial Chemical Industries PLC Legal Department: Patents Thames House North Millbank, London SW1P 4QG (GB)**

(54) **Fluorobenzyl cyclopropane carboxylates, their preparation, compositions and use as insecticides.**

(57) Fluorobenzyl esters of tetramethylcyclopropane carboxylic acid in which the phenyl ring may also be substituted with halo, alkyl, alkenyl, alkoxy, alkylthio, amino, alkoxyalkyl or alkylaminoalkyl groups. A typical example is pentafluorobenzyl, 2,2,3,3-tetramethylcyclopropane carboxylate. The compounds and their compositions are useful as insecticides.

EP 0 060 617 A1

# FLUOROBENZYL CYCLOPROPANE CARBOXYLATES, THEIR PREPARATION, COMPOSITIONS AND USE AS INSECTICIDES

This invention relates to novel cyclopropane derivatives useful as insecticides, to processes for their preparation, to compositions comprising them and to methods of combatting insect and similar invertebrate pests using them.

Certain naturally occurring esters of cyclopropane carboxylic acids have long been known to possess insecticidal properties, but these compounds have been too easily degraded by ultra violet light to be of much use in agriculture. Several groups of synthetic compounds based on cyclopropane carboxylic acids (for example those disclosed in British patent specifications nos. 1,243,858 and 1,413,491) have been evaluated in an attempt to discover compounds of sufficient light stability for use as general agricultural insecticides. In particular some chlorobenzyl esters of tetramethylcyclopropane carboxylic acid are described in British Patent Specification No. 1178857 but their insecticidal activity towards some important pests of agriculture is not very satisfactory.

We have discovered that compounds of this type in which there is at least one fluoro substituent in the benzyl group have improved insecticidal performance.

Accordingly the present invention provides fluoro benzyl esters of tetramethylcyclopropane carboxylic acid, and in particular provides compounds of formula :

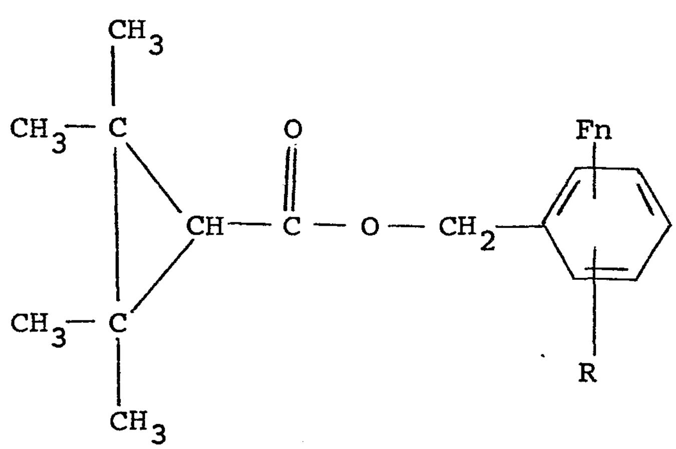

(I)

Wherein n represents an integer from one to four and R represents hydrogen, halo, alkyl of up to four carbon atoms, alkenyl of up to five carbon atoms, benzyl, alkoxy of up to four carbon atoms, alkenyloxy of up to five carbon atoms, alkylthio of up to four carbon atoms, alkane sulphonyl of up to four carbon atoms, amino, acylamino of up to four carbon atoms, alkylamino of up to four carbon atoms, dialkylamino of up to a total of eight carbon atoms, N-(alkyl)acylamino of up to a total of eight carbon atoms, alkoxyalkyl of up to a total of six carbon atoms, aryloxyalkyl of up to a total of nine carbon atoms, alkenyloxyalkyl of up to a total of six carbon atoms, alkylthioalkyl of up to a total of six carbon atoms, alkylaminoalkyl of up to a total of six carbon atoms, or dialkylaminoalkyl of up to a total of ten carbon atoms.

Thus R may be, for example, hydrogen, fluoro, chloro, bromo, methyl, ethyl, propyl, butyl, allyl, butenyl, methylbutenyl, benzyl, methoxy, ethoxy, allyloxy, ethylthio, ethanesulphonyl, amino, acetamido, diethylamino, N-methylacetamido, methoxymethyl, ethoxymethyl, propoxymethyl, phenoxymethyl, allyloxymethyl, methylthiomethyl, ethylthiomethyl, or diethylaminomethyl.

A first preferred group of compounds is that wherein n is four, especially when R is 4-fluoro, 4-methyl, 4-methoxy, 4-methoxymethyl or 4-allyl group. A second preferred group of compounds is that wherein n is one and R represents halo, especially 2-chloro.

Specific compounds according to the invention include those set out in Table I below which correspond to the general formula IA.

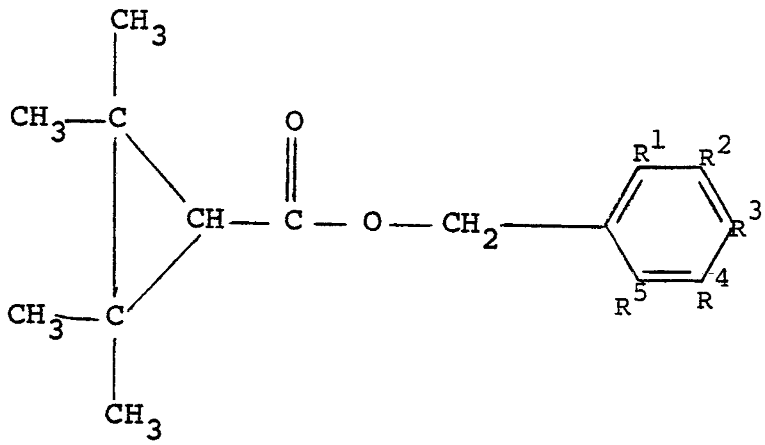

TABLE I

| COMPOUND NO | $R^1$ | $R^2$ | $R^3$ | $R^4$ | $R^5$ |
|---|---|---|---|---|---|
| 1 | F | F | F | F | F |
| 2 | F | F | $CH_3$ | F | F |
| 3 | F | F | $OCH_2CH=CH_2$ | F | F |
| 4 | F | F | $OCH_3$ | F | F |
| 5 | F | F | $CH_2OCH_3$ | F | F |
| 6 | Cl | H | H | H | F |
| 7 | Cl | H | F | H | H |
| 8 | F | F | $SC_2H_5$ | F | F |
| 9 | F | F | $SO_2C_2H_5$ | F | F |
| 10 | $CH_3$ | F | F | F | F |
| 11 | F | F | $OC_2H_5$ | F | F |
| 12 | F | F | $C_2H_5$ | F | F |
| 13 | F | F | $OCH_2CH=CH_2$ | F | F |
| 14 | F | F | $\underline{n}-C_4H_9$ | F | F |
| 15 | F | $CH_3$ | F | F | F |
| 16 | F | F | $\underline{n}-C_3H_7$ | F | F |
| 17 | F | F | $CH_2CH=C(CH_3)_2$ | F | F |
| 18 | F | F | $CH_2CH=CHCH_3$ | F | F |
| 19 | F | F | $CH_2C_6H_5$ | F | F |
| 20 | F | F | $NHCOCH_3$ | F | F |
| 21 | F | F | $N(CH_3)COCH_3$ | F | F |
| 22 | F | F | $N(C_2H_5)_2$ | F | F |

TABLE I CONTINUED

| COMPOUND NO | $R^1$ | $R^2$ | $R^3$ | $R^4$ | $R^5$ |
|---|---|---|---|---|---|
| 23 | F | F | $NH_2$ | F | F |
| 24 | F | H | $CH_2CH=CH_2$ | H | F |
| 25 | H | F | $CH_2CH=CH_2$ | F | H |
| 26 | F | H | $OCH_3$ | H | F |
| 27 | F | F | $CH_2OC_2H_5$ | F | F |
| 28 | F | F | $CH_2O$-$\underline{n}$-$C_3H_7$ | F | F |
| 29 | F | F | $CH_2OC_6H_5$ | F | F |
| 30 | F | F | $CH_2SC_2H_5$ | F | F |
| 31 | F | F | $CH_2OCH_2CH=CH_2$ | F | F |
| 32 | F | F | H | F | F |
| 33 | F | F | Cl | F | F |
| 34 | F | F | Br | F | F |
| 35 | H | F | F | F | F |
| 36 | F | F | F | H | F |
| 37 | F | F | $CH_2N(C_2H_5)_2$ | F | F |
| 38 | H | F | Cl | F | F |
| 39 | H | H | H | F | F |
| 40 | F | H | H | H | F |
| 41 | H | F | H | H | F |
| 42 | F | H | H | H | H |
| 43 | H | H | F | H | H |
| 44 | H | F | H | H | H |
| 45 | F | F | $CH_2SCH_3$ | F | F |

The compounds of the invention are esters and may be prepared by conventional esterification processes, such as those described in British patent no. 1,178,857. A particularly useful process in that wherein an acid halide (especially the acid chloride) of tetramethylcyclopropane carboxylic acid is reacted with an alcohol of formula

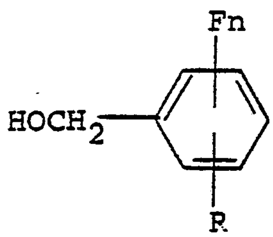

II

where n and R have any of the meanings given hereinabove in the presence of a hydrogen halide acceptor such as a tertiary organic base such as pyridine or an alkali or alkaline earth metal carbonate, preferably in the presence of an inert diluent such as a lower aliphatic ketone or a halohydrocarbon, e.g. methylene chloride at a temperature within the range 0 to 100°C preferably 25 to 30°C.

Many of the compounds of formula II, particularly those wherein n is four and R is alkyl, alkenyl, alkoxy, alkenyloxy or alkoxyalkyl, or similar groups such as alkylthio, alkylsulphonyl, amino, alkylamino, benzyl, alkylthioalkyl or alkylaminoalkyl, have not previously been described.

The compounds of formula II may be prepared by different processes depending upon the nature of the substituents in the benzene ring. Thus for alkyl- or alkenyl- substituted compounds of formula II where R is alkyl the appropriately substituted alkyl or alkenyl-fluorobenzene may be carboxylated (for example by the use of an organometallic reagent such as alkyl lithium, followed by decomposition of the reaction product with carbon dioxide) and subsequent reduction to the alcohol, using an appropriate reducing reagent, for example, lithium aluminium hydride.

The alkyl- or alkenyl- substituted fluorobenzenes used as starting materials in this sequence may be prepared by the alkylation of the appropriate fluorobenzenes using organometallic reagents such as alkyl lithium, and decomposing the reaction products with alkyl or alkenyl halides.

Alternatively the fluorobenzenes may be carboxylated first, and the resultant fluorobenzoic acids reduced to the benzyl alcohol which is then alkylated or alkenylated in a protected form (for example as the tetrahydropyranyl ether) using alkyllithium followed by reaction with an alkyl or alkenyl halide.

All of these processes are illustrated in the following scheme.

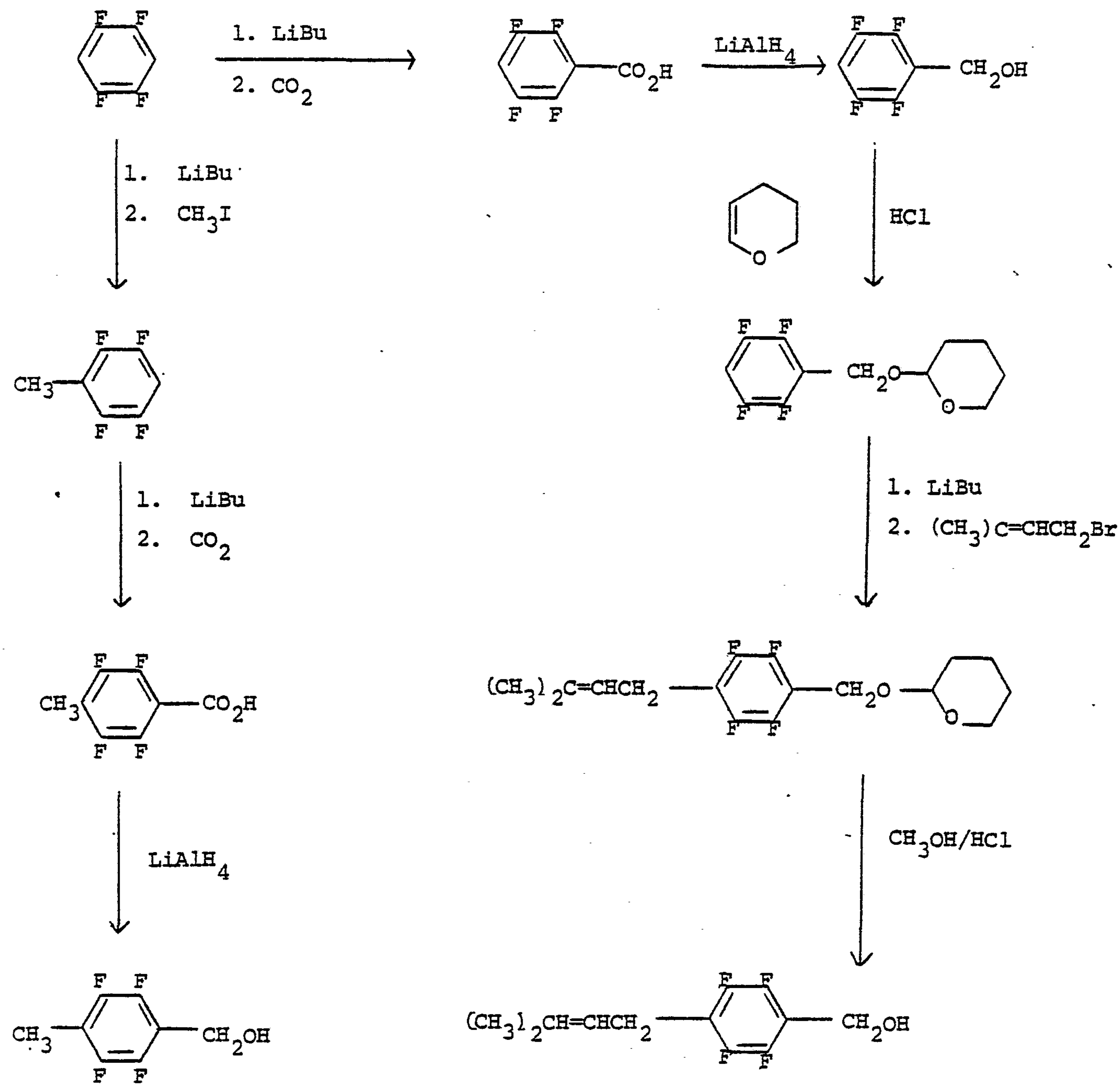


The compounds of formula II wherein R is alkylthio or alkoxy may be prepared by displacement of halogen, e.g. fluorine, from an appropriately substituted fluorobenzyl alcohol, or the tetrahydropyranyl ether thereof. The following scheme illustrates the reactions used to prepare a number of compounds of formula II.

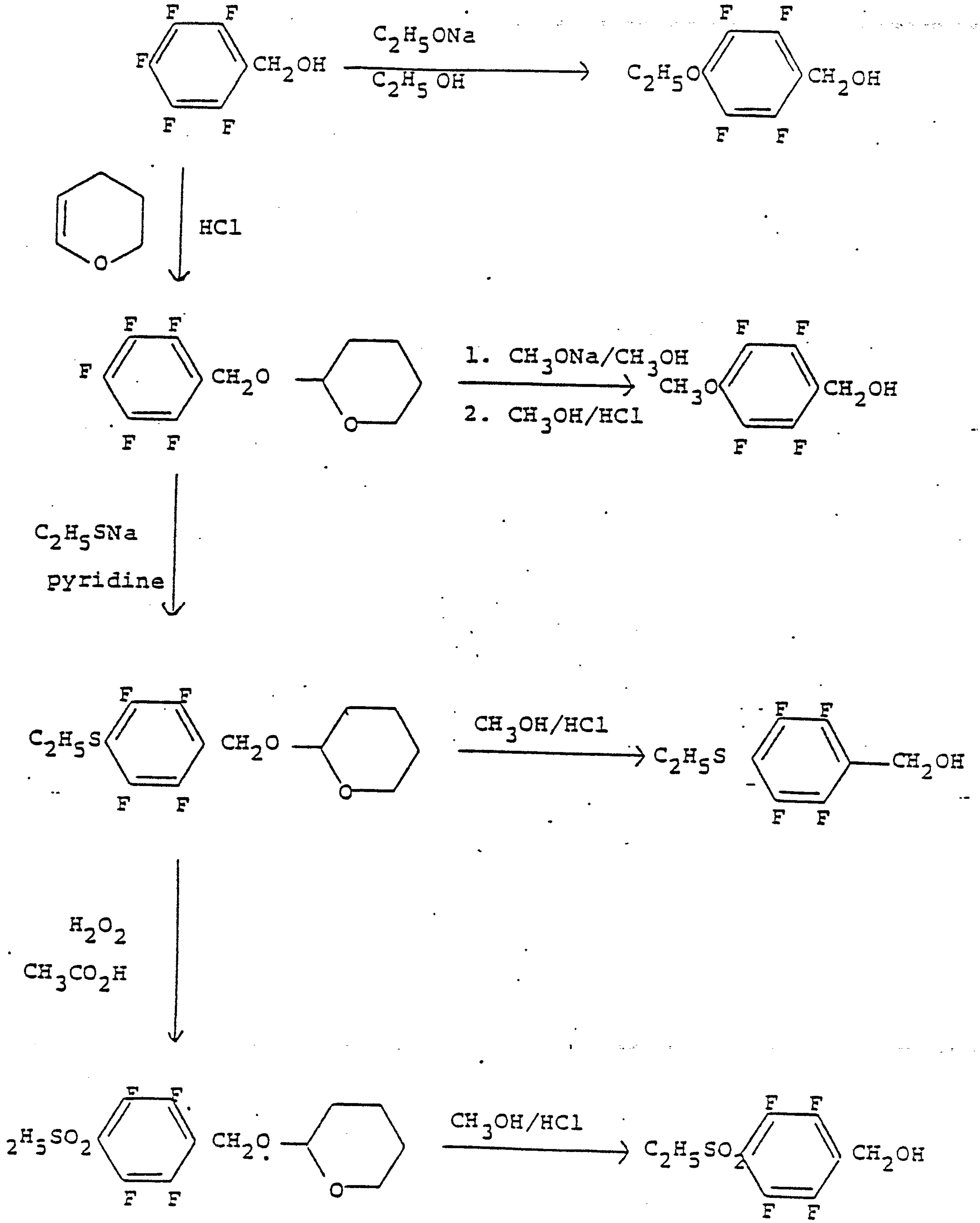
C2H5ONa
C2H5 OH
HCl
1. CH3ONa/CH3OH
2. CH3OH/HCl
C2H5SNa
pyridine
CH3OH/HCl
H2O2
CH3CO2H
CH3OH/HCl

Similarly the compounds of formula II where R is amino or substituted amino may also be obtained from the corresponding fluorobenzyl alcohol. The following scheme illustrates the reactions involved in preparing some of these compounds.

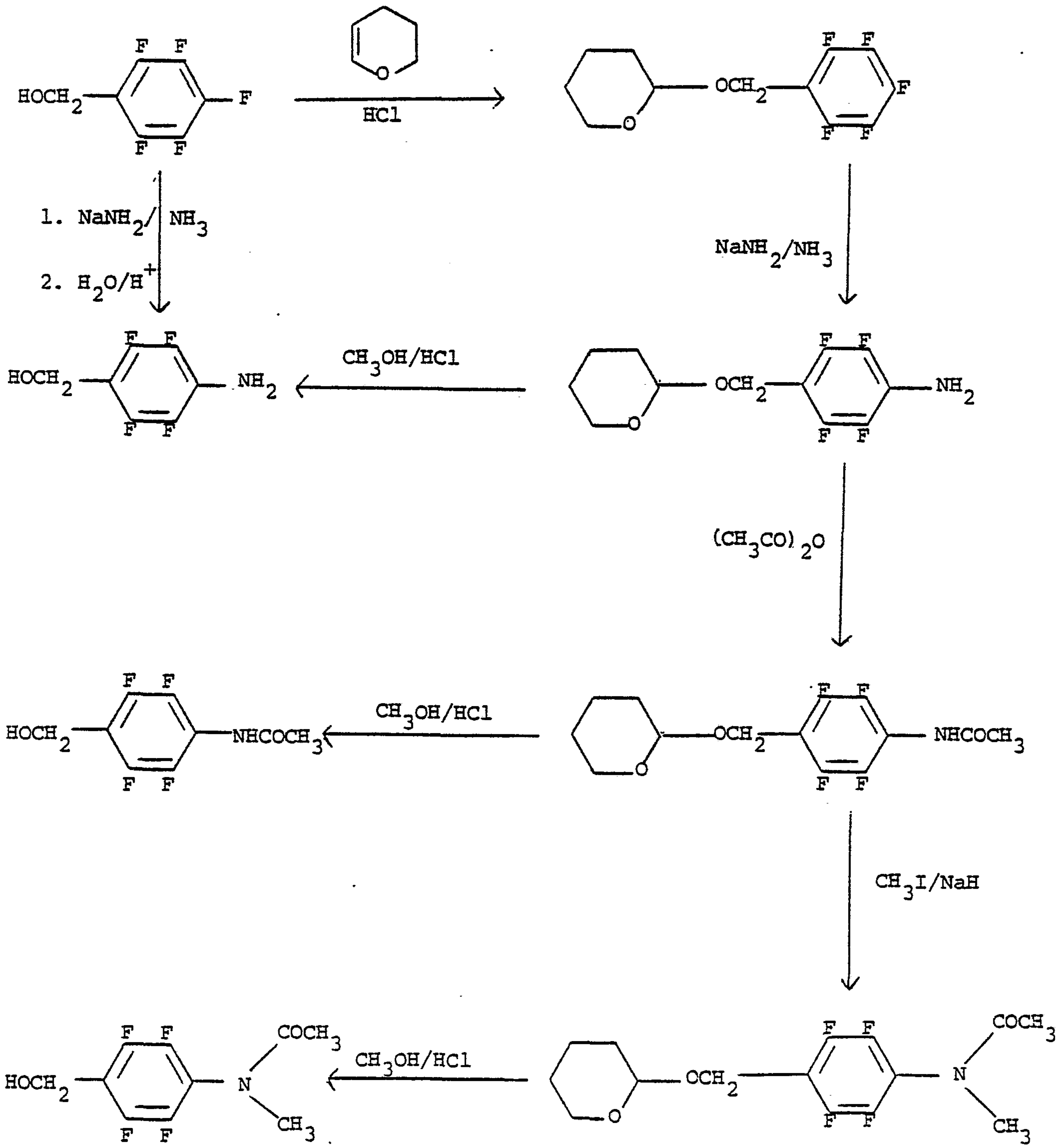

In an alternative process compounds of formula I where R represents an acylamino group may be obtained by acylation (e.g. by reaction with the appropriate acyl chloride) of the corresponding compound where $R^4$ is hydrogen.

The compounds of formula II wherein R represents an alkoxyalkyl, alkylthioalkyl or alkylaminoalkyl group may be obtained by reactions similar to those illustrated in the following Scheme.

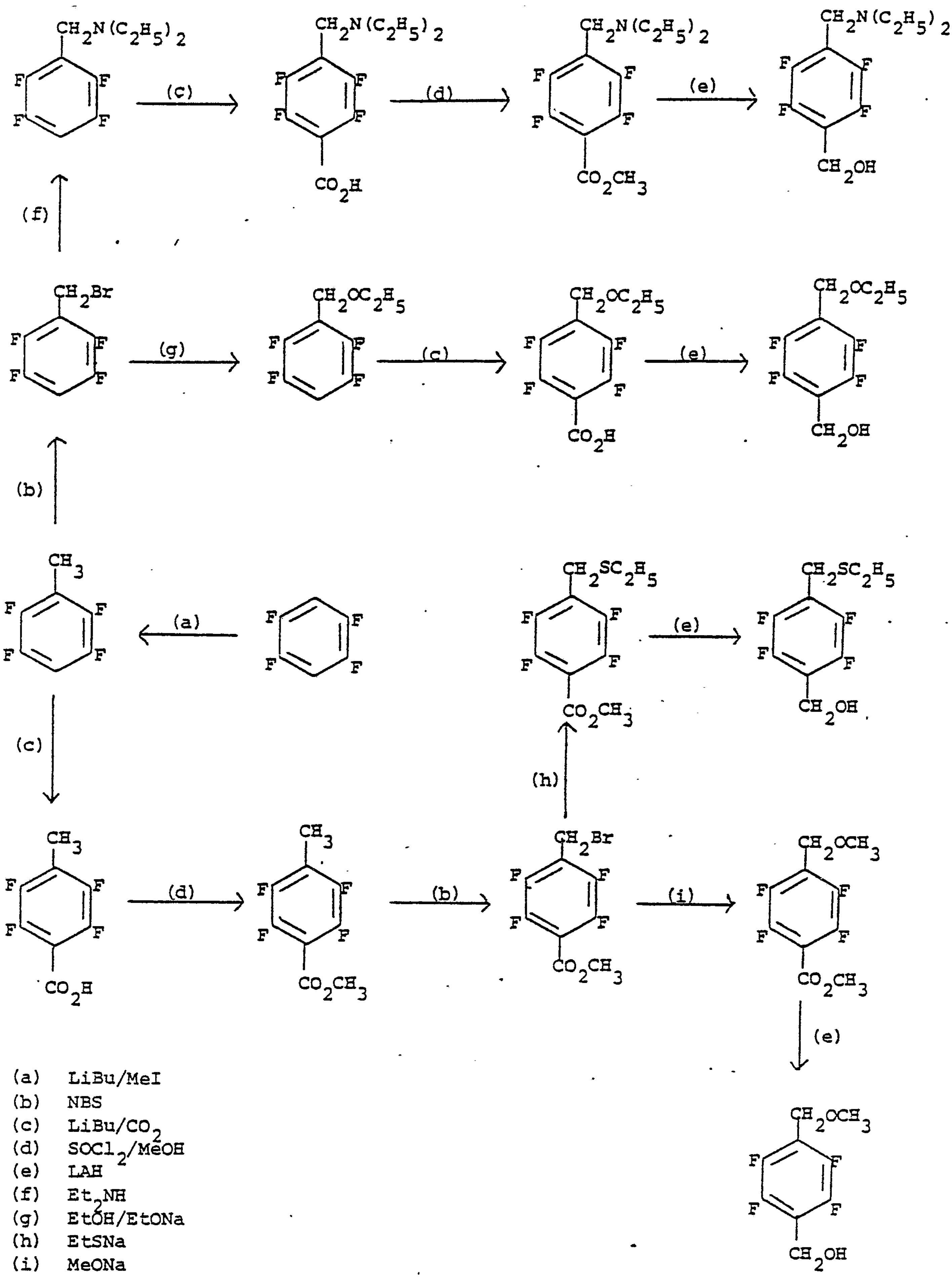
(a) LiBu/MeI
(b) NBS
(c) $LiBu/CO_2$
(d) $SOCl_2/MeOH$
(e) LAH
(f) $Et_2NH$
(g) EtOH/EtONa
(h) EtSNa
(i) MeONa

The compounds of formula I may be used to combat and control infestations of insect pests and also other invertebrate pests, for example, acarine pests. The insect and acarine pests which may be combatted and controlled by the use of the invention compounds include those pests associated with agriculture (which term includes the growing of crops for food and fibre products, horticulture and animal husbandry), forestry, the storage of products of vegetable origin, such as fruit, grain and timber, and also those pests associated with the transmission of diseases of man and animals.

In order to apply the compounds to the locus of the pests they are usually formulated into compositions which include in addition to the insecticidally active ingredient or ingredients of formula I suitable inert diluent or carrier materials, and/or surface active agents. The compositions may also comprise another pesticidal material, for example another insecticide or acaricide, or a fungicide, or may also comprise a insecticide synergist, such as for example dodecyl imidazole, safroxan, or piperonyl butoxide.

The compositions may be in the form of dusting powders wherein the active ingredient is mixed with a solid diluent or carrier, for example kaolin, bentonite, kieselguhr, or talc, or they may be in the form of granules, wherein the active ingredient is absorbed in a porous granular material for example pumice, gypsum or corn cob grits. Granules are particularly useful for combating soil borne insect pests, such as root worms of the genus Diabrotica, cutworms (Agrotis spp.) and wireworms (Agriotis spp.). Preferably, the granules contain from 1 to 2.5% by weight of the active ingredient, which is absorbed onto the granule by, for example, spraying the granules with a solution of the active ingredient in a volatile solvent which is subsequently evaporated from the surface of the granules. Such solutions may contain other ingredients, for example a resin to regulate the rate of release of the active

ingredient from the granules, or to help prevent premature disintegration of the granules. Granules may be applied to the soil either in a band between the furrows defining the crop rows, or broadcast, and may if desired be lightly incorporated in the soil, or they may be placed in the furrows themselves at the time of planting the crop. Application of granules at a rate of from 5 to 25 lb/acre (approximately 5 to 25 kg/ha) is usually sufficient to control the pests, and a preferred rate is within the range 8 to 15 lb/acre (approximately 8 to 15 kg/ha) based on the active ingredient.

Alternatively the compositions may be in the form of liquid preparations to be used as dips or sprays, which are generally aqueous dispersions or emulsions of the active ingredient in the presence of one or more known wetting agents, dispersing agents or emulsifying agents (surface active agents).

Wetting agents, dispersing agents and emulsifying agents may be of the cationic, anionic or non-ionic type. Suitable agents of the cationic type include, for example, quaternary ammonium compounds, for example cetyltrimethyl ammonium bromide. Suitable agents of the anionic type include, for example, soaps, salts of aliphatic monoesters or sulphuric acid, for example sodium lauryl sulphate, salts of sulphonated aromatic compounds, for example sodium dodecylbenzenesulphonate, sodium, calcium or ammonium lignosulphonate, or butylnaphthalene sulphonate, and a mixture of the sodium salts of diisopropyl- and triisopropylnaphthalene sulphonates. Suitable agents of the non-ionic type include, for example, the condensation products of ethylene oxide with fatty alcohols such as oleyl alcohol or cetyl alcohol, or with alkyl phenols such as octyl phenol, nonyl phenol and octyl cresol. Other non-ionic agents are the partial esters derived from long chain fatty acids and hexitol anhydrides, the condensation products of the said partial esters with ethylene oxide, and the lecithins.

The compositions may be prepared by dissolving the active ingredient in a suitable solvent, for example, a ketonic solvent such as diacetone alcohol, or an aromatic solvent such as trimethylbenzene and adding the mixture so obtained to water which may contain one or more known wetting, dispersing or emulsifying agents. Other suitable organic solvents are dimethyl formamide, ethylene dichloride, isopropyl alcohol, propylene glycol and other glycols, diacetone alcohol, toluene, kerosene, white oil, methylnaphthalene, xylenes and trichloroethylene, N-methyl-2-pyrrolidone and tetrahydro furfuryl alcohol (THFA).

The compositions to be used as sprays may also be in the form of aerosols wherein the formulation is held in a container under pressure in the presence of a propellant such as fluorotrichloromethane or dichlorodifluoromethane.

The compositions which are to be used in the form of aqueous dispersions or emulsions are generally supplied in the form of a concentrate containing a high proportion of the active ingredient or ingredients, the said concentrate to be diluted with water before use. These concentrates are often required to withstand storage for prolonged periods and after such storage, to be capable of dilution with water to form aqueous preparations which remain homogenous for a sufficient time to enable them to be applied by conventional spray equipment. The concentrates may contain 10-85% by weight of the active ingredient or ingredients. When diluted to form aqueous preparations such preparations may contain varying amounts of the active ingredient depending upon the purpose for which they are to be used. For agricultural or horticultural purposes, an aqueous preparation containing between 0.0001% and 0.1% by weight of the active ingredient is particularly useful.

In use the compositions are applied to the pests, to the locus of the pests, to the habitat of the pests, or to growing plants liable to infestation by the pests, by any of the known means of applying pesticidal compositions, for example, by dusting or spraying.

The compositions of the invention are very toxic to wide varieties of insect and other invertebrate pests, including, for example, the following:-

*Aphis fabae* (aphids)
*Megoura viceae* (aphids)
*Aedes aegypti* (mosquitoes)
*Dysdercus fasciatus* (capsids)
*Musca domestica* (houseflies)
*Pieris brassicae* (white butterfly, larvae)
*Plutella maculipennis* (diamond back month, larvae)
*Phaedon cochleariae* (mustard beetle)
*Telarius cinnabarinus* (carmine spider mite)
*Aonidiella* spp. (scale insects)
*Trialeuroides* spp. (white flies)
*Blattella germanica* (cockroaches)
*Spodoptera littoralis* (cotton leaf worm)
*Chortiocetes terminifera* (locusts)
*Diabrotica* spp. (rootworms)
*Agrotis* spp. (cutworms)

The compounds of formula I and compositions comprising them have shown themselves to be particularly useful in controlling lepidopteran pests of cotton, for example *Spodoptera* spp. and *Heliothis* spp. The fumigant properties of the compounds enable them to be used to combat pests which inhabit the soil, for example *Diabrotica* spp. They are also excellent knock down agents and as such may be used in conjunction with other insecticides to combat public health pests such as flies. They are also very useful in combatting insect and acarine pests which infest domestic animals, such as *Lucilia sericata*, and ixodid ticks such as *Boophilus* spp., *Ixodes* spp., *Amblyomma* spp., *Rhipicephalus* spp., and *Dermaceutor* spp. They are effective in combatting both susceptible and resistant strains of these pests in their adult, larval and intermediate stages of growth, and may be applied to the infested host animal by topical, oral or parenteral administration.

The following Examples illustrate the various aspects of the invention.

EXAMPLE 1

This Example illustrates the insecticidal properties of compounds of this invention.

The activity of the compounds was determined using a variety of insect pests. The compound was used in the form of liquid preparations containing 500, 100, 50 or 25 parts per million (p.p.m.) by weight of the compound. The preparations were made by dissolving the compound in a mixture of solvents consisting of 4 parts by volume of acetone and 1 part by volume of diacetone alcohol. The solutions were then diluted with water containing 0.01% by weight of a wetting agent sold under the trade name "LISSAPOL" NX until the liquid preparations contained the required concentration of the compound. "Lissapol" is a Registered Trade Mark.

The test procedure adopted with regard to each pest was basically the same and comprised supporting a number of the pests on a medium which was usually a host plant or a foodstuff on which the pests feed, and treating either or both the pests and the medium with the preparations. The mortality of the pests was then assessed at periods usually varying from one to three days after the treatment. Details are given in Table II.

The results of the tests are given in Table III for each of the compounds at the rate in parts per million given in the second column as a grading of mortality on a scale of 0-9 wherein

| | | | | |
|---|---|---|---|---|
| 0 | represents | less than | 10% | mortality |
| 1 | " | from | 10 to 19% | " |
| 2 | " | " | 20 to 29% | " |
| 3 | " | " | 30 to 39% | " |
| 4 | " | " | 40 to 49% | " |
| 5 | " | " | 50 to 59% | " |
| 6 | " | " | 60 to 69% | " |
| 7 | " | " | 70 to 79% | " |
| 8 | " | " | 80 to 89% | " |
| 9 | " | " | 90 to 100% | " |

In Table III the pest organism used is designated by a letter code and the pest species, the support medium or food, and the type and duration of test is given in Table II.

TABLE II

| CODE LETTERS (Table III) | PEST SPECIES | SUPPORT MEDIUM/FOOD | TYPE OF TEST* | DURATION (days) |
|---|---|---|---|---|
| MD | Musca domestica (houseflies - adults) | Cotton wool/ milk, sugar | Contact | 1 |
| SL | Spodoptera littoralis (cotton leaf worm - larvae) | Cabbage leaves | Residual | 3 |
| PX | Plutella xylostella (diamond back moth - larvae) | Cabbage leaves | Residual | 3 |
| HA | Heliothis armigera (cotton boll worm - larvae) | Standard synthetic rearing medium | Contact | 3 |
| DB | Diabrotica balteata (rootworm - larvae) | Filter paper | Contact | 3 |

* "Contact" test indicates that both pests and medium were treated and "residual" indicates that the medium was treated before infestation with the pests.

TABLE III

| PRODUCT | RATE (ppm) | PEST SPECIES | | | | |
|---|---|---|---|---|---|---|
| | | MD | SL | PX | HA | DB |
| 1 | 100 | 8 | 9 | 0 | 8 | 9 |
| 2 | 100 | 9 | - | 9 | 6 | 9 |
| 3 | 50 | 9 | 9 | 9 | 9 | 9 |
| 4 | 100 | 9 | 9 | 9 | 8 | 9 |
| 5 | 100 | 9 | 9 | 9 | 9 | 9 |
| 6 | 100 | 3 | 0 | 0 | 0 | 9 |
| 7 | 100 | 6 | 0 | 0 | 0 | 9 |
| 37 | 100 | 0 | 0 | 9 | - | 9 |
| 45 | 100 | 9 | 2 | 5 | - | 9 |

A dash (-) in Table IV above indicates that the compound had not been tested against the particular pest species.

In further tests the compounds showed insecticidal activity against a number of other species. Thus for example compound no.3 showed good control of Aphis fabae, red spider mite adults (Tetranychus urticae, organophosphate resistant strain) on French bean leaves, and plant hoppers (Nilaparvata lugens) on rice.

EXAMPLE 2

This Example illustrates the preparation of 2-methyl-3,4,5,6-tetrafluorobenzyl alcohol.

(a) Preparation of 2,3,4,5-tetrafluorotoluene.

A solution of n-butyllithium in hexane (1.6M, 62.5 ml) was added dropwise to a well stirred solution of 1,2,3,4-tetrafluorobenzene (15.0g) in dry tetrahydrofuran (150 ml) maintained at a temperature of -60°C under an atmosphere of dry argon. When the addition was complete the mixture was stirred at -45°C for 2 hours and then methyl iodide (14.2g) was added dropwise whilst the temperature was kept at -45°C. After a period of 30 minutes the mixture was allowed to warm to the ambient temperature, poured into distilled water and the mixture extracted with diethyl ether (2 x 50 ml), and the extracts dried over anhydrous magnesium sulphate. After filtering the solution was concentrated by evaporation of the solvents at atmospheric pressure. The residual oil was distilled and the fraction boiling in the range 115-122°C at atmospheric pressure 6.2 g) collected, identified by n.m.r. and gas chromatographic analysis as consisting of ca. 90% of the required 2,3,4,5-tetrafluorotoluene and ca. 10% of 3,4,5,6-tetrafluoro-1,2-xylene.

(b) Preparation of 3,4,5,6-tetrafluoro-2-toluic acid.

The product of step (a) above (5.5g) was mixed with diethyl ether (35 ml), the mixture cooled to -70°C, and maintained at this temperature whilst a solution of n-butyllithium in n-hexane (1.6M, 21 ml) was slowly added.

The mixture was stirred for a period of 1 hour during which time a fine white precipitate was formed. Dry carbon dioxide gas was then passed into the mixture for 30 minutes whilst the temperature was maintained within the range -70°C to -40°C, and continued to be passed in thereafter whilst the mixture was allowed to warm to the ambient temperature. After acidifying with dilute hydrochloric acid (6N, 40 ml) the organic phase was separated, washed with water and dried over anhydrous magnesium sulphate. After evaporation of the solvents under reduced pressure the residual oil (which from n.m.r. analysis was shown to be an approximately 1:1 mixture of the desired product and pentanoic acid) was carefully distilled under reduced pressure (water pump) using a Kugelrohr apparatus, and the fraction which solidified on cooling collected and recrystallised from toluene to yield 3,4,5,6-tetrafluoro-2-toluic acid, m.p. 165°C (0.65g), identified by infra red and nuclear magnetic resonance spectroscopy.

(c) Preparation of 2-methyl-3,4,5,6-tetrafluorobenzyl alcohol.

3,4,5,6-tetrafluoro-2-toluic acid (500 mg) was dissolved in dry diethyl ether (5.0 ml) added dropwise to a suspension of lithium aluminium hydride (100 mg) in dry ether (10 ml) under an argon atmosphere at the ambient temperature. When the addition was complete and the effervescence had subsided the mixture was heated at the reflux temperature for a period of 1 hour. After allowing the mixture to cool to the ambient temperature water (10 ml) was cautiously added and the resultant mixture extracted with diethyl ether (2 x 20 ml), the extracts

washed with water and dried over anhydrous magnesium sulphate. After filtration the filtrate was concentrated by evaporation of the solvent. The residual low melting solid was recrystallised from petroleum ether (boiling range 40-60°C) to yield 2-methyl-3,4,5,6-tetrafluorobenzyl alcohol (200 mg). N.m.r. ('H(ppm)$CDCl_3$): 2.18(s,1H); 2.32(t,3H); 4.86(s,2H).

EXAMPLE 3

The procedures of Example 2 were used to prepare various benzyl alcohols from tetrafluorobenzenes via the appropriate benzoic acids.

(a) By the procedure set out in paragraph (a) of Example 2 (but using the appropriate lithium derivatives) the following conversions were effected:

(a)(i) 1,2,4,5-tetrafluorobenzene to 2,3,5,6-tetrafluorotoluene (b.p. 117-121°C, contaminated with ca 5% 2,3,5,6-tetrafluoroxylene).
N.m.r. ($^1H$(ppm)$CDCl_3$): 2.28(t,3H); 6.58-6.94 (m,1H). Infra red (liquid film): 3075, 1645, 1510, 1255, 1165 $cm^{-1}$.

(ii) 1,2,4,5-tetrafluorobenzene to ethyl-2,3,5,6-tetrafluorobenzene (b.p. 128-134°C).
N.m.r. ($^1H$(ppm)$CDCl_3$): 1.20 (t,3H); 2.72 (q,2H);
Infra red (liquid film): 3075, 1650, 1510, 1250 $cm^{-1}$.

(iii) 1,2,4,5-tetrafluorobenzene to <u>n</u>-propyl-2,3,5,6-tetrafluorobenzene (b.p. 142-146°C).
N.m.r. ($^1H$(ppm)$CDCl_3$): 0.96 (t,3H); 1.64 (q,2H); 2.74 (t,2H); 6.74-7.08 (m,1H)
Infra red (liquid film): 3075, 1655, 1495, 1255 $cm^{-1}$

(iv) 1,2,4,5-tetrafluorobenzene to benzyl-2,3,5,6-tetrafluorobenzene (m.p. 38-40°C)
N.m.r. ($^1$H(ppm)$CDCl_3$): 4.02 (s,2H); 6.68-7.08 (m,1H); 7.20 (s,5H).
Infra red (liquid paraffin): 3080, 1645, 1605, 1500 1250 $cm^{-1}$

(v) 1,2,4,5-tetrafluorobenzene to allyl-2,3,5,6-tetrafluorobenzene
N.m.r. ($^1$H(ppm)$CDCl_3$): 3.40 (m,2H); 4.78-5.18 (m,2H); 5.60-6.05 (m,1H); 6.60-7.00 (m,1H).
Infra red (liquid film): 3080, 1640, 1500, 1250, 1170, 850 $cm^{-1}$

(b) By the use of the procedure set out in paragraph (b) of Example 2 the following benzoic acids were obtained from the appropriate precursor as follows:

(i) 4-methyl-2,3,5,6-tetrafluorobenzoic acid (from 2,3,5,6-tetrafluorotoluene) - m.p. 170°C.
N.m.r. ($^1$H(ppm)$CDCl_3$): 2.44(t,3H); 11.56 (s,1H)
Infra red (liquid paraffin): 3300-2450, 1710, 1650, 1460, 1070 $cm^{-1}$.

(ii) 4-ethyl-2,3,5,6-tetrafluorobenzoic acid (from ethyl-2,3,5,6-tetrafluorobenzene) - m.p. 92-93°C.
N.m.r. ($^1$H(ppm)$CDCl_3$): 1.24 (t,3H); 2.80 (q,2H) 13.30 (s,1H).
Infra red (liquid paraffin): 3300-2450, 1710, 1650, 1485, 1460, 965 $cm^{-1}$

(iii) 4-*n*-propyl-2,3,5,6-tetrafluorobenzoic acid (from *n*-propyl 2,3,5,6-tetrafluorobenzene) - m.p. 65-68°C.
N.m.r. ($^1$H(ppm)$CDCl_3$): 0.98 (t,3H); 1.68 (q,2H) 2.76 (t,2H); 11,34 (s,1H)
Infra red (liquid paraffin): 3300-2450, 1710, 1650, 1485, 1450 $cm^{-1}$

(iv) 4-benzyl-2,3,5,6-tetrafluorobenzoic acid (from benzyl-2,3,5,6-tetrafluorobenzene) - m.p. 161-164°C
N.m.r. ($^1H$(ppm)$CDCl_3$): 4.06 (s,2H); 7.22 (s,5H) 10.06 (s,1H)
Infra red (liquid paraffin): 3300-2450, 1705, 1650, 1485, 1005 $cm^{-1}$

(v) 4-allyl-2,3,5,6-tetrafluorobenzoic acid (from allyl-2,3,5,6-tetrafluorobenzene) - m.p. 88-90°C
N.m.r. ($^1H$(ppm)$CDCl_3$): 3.50 (m,2H); 4.95-5.20 (m,2H); 5.60-6.08 (m,1H); 11.82 (s,1H)
Infra red (liquid paraffin): 3300-2300, 1700, 1650, 1470, 1410, 1290, 1240, 980 $cm^{-1}$

(c) By the procedure set out in paragraph (c) of Example 2 the benzyl alcohols were obtained by reduction of the appropriate acids as follows:

(i) 4-methyl-2,3,5,6-tetrafluorobenzyl alcohol (from 4-methyl-2,3,5,6-tetrafluorobenzoic acid) - m.p. 61-62°C.
N.m.r. ($^1H$(ppm)$CDCl_3$): 2.24 (t,3H); 2.06 (s,1H) 4.82 (s,2H)
Infra red (liquid paraffin): 3300-1660, 1460, 1280, 1020 $cm^{-1}$

(ii) 4-ethyl-2,3,5,6-tetrafluorobenzyl alcohol (from 4-ethyl-2,3,5,6-tetrafluorobenzoic acid) - m.p. 36-37°C
N.m.r. ($^1H$(ppm)$CDCl_3$): 1.22 (t,3H); 2.06 (s,1H); 2.76 (q,2H); 4.78 (s,2H)
Infra red (liquid paraffin): 3300, 1660, 1490, 1465, 1280 $cm^{-1}$

(iii) 4-*n*-propyl-2,3,5,6-tetrafluorobenzyl alcohol (from 4-*n*-propyl-2,3,5,6-tetrafluorobenzoic acid).

0060617

N.m.r. ($^{1}H$(ppm)$CDCl_3$): 0.94 (t,3H); 1.60 (q,2H); 2.12 (s,1H); 2.66 (t,2H); 4.70 (s,2H)

Infra red (liquid film): 3350, 1660, 1485, 1280, 1015 $cm^{-1}$

(iv) 4-benzyl-2,3,5,6-tetrafluorobenzyl alcohol (from 4-benzyl-2,3,5,6-tetrafluorobenzoic acid) - m.p. 72-74°C.
N.m.r. ($^{1}H$(ppm)$CDCl_3$): 2.00 (s,1H); 4.04 (s,2H); 4.78 (s,2H); 7.22 (s,5H)
Infra red (liquid paraffin): 3300, 1655, 1485, 1010 $cm^{-1}$

(v) 4-allyl-2,3,5,6-tetrafluorobenzyl alcohol (from 4-allyl-2,3,5,6-tetrafluorobenzoic acid)
N.m.r. ($^{1}H$(ppm)$CDCl_3$): 2.42 (s,1H); 3,35 (m,2H) 4.68 (m,2H); 4.82-5.10 (m,2H); 5.55-5.98 (m,1H)
Infra red (liquid film): 3600-3100, 2950, 1640, 1490 1300, 1270, 1020, 860 $cm^{-1}$

EXAMPLE 4

This Example illustrates the preparation of 4-methoxy-2,3,5,6-tetrafluorobenzyl alcohol.

Pentafluorobenzyl alcohol (1.98 g) was added to a stirred solution of sodium methoxide (obtained by dissolving sodium (0.4 g) in methyl alcohol (10 ml) at the ambient temperature, and the mixture heated at the reflux temperature for 3.5 hours. After cooling the mixture, the solvent was removed by evaporation under reduced pressure and the residue partitioned between water and diethyl ether. After separating the ethereal layer and washing with water, it was dried over anhydrous magnesium sulphate and concentrated by evaporation of the solvent under

reduced pressure to yield 4-methoxy-2,3,5,6-tetrafluorobenzyl alcohol (1.8 g), identical with the product of Example 5.

Infra red (liquid film): 3600-3100, 2950, 1650, 1500, 1200, 1130, 1040, 1000, 930cm$^{-1}$

EXAMPLE 5

This Example illustrates the preparation of 4-methoxy-2,3,5,6-tetrafluorobenzyl alcohol.

(a) Preparation of pentafluorobenzyl tetrahydropyran-2-yl ether.

A mixture of pentafluorobenzyl alcohol (17.87 g) 2,3-dihydro-4H-pyran (8.3 g), dry diethyl ether (100 ml) and concentrated hydrochloric acid (0.2 ml) was stirred at the ambient temperature for 4 hours, after which the mixture was washed with water, dried over anhydrous magnesium sulphate and concentrated by evaporation under reduced pressure. The residual oil was identified by infra red spectroscopy as pentafluorobenzyl tetrahydropyran-2-yl ether.

(b) Preparation of 4-methoxy-2-3,5,6-tetrafluorobenzyl tetrahydropyran-2-yl ether.

To a mixture of freshly prepared dry sodium methoxide (obtained by dissolving sodium metal (0.46 g) in dry methyl alcohol and evaporating to dryness) and dry pyridine (25 ml) was added, dropwise with vigorous stirring, a solution of pentafluorobenzyl tetrahydropyran-2-yl ether (2.82 g) in dry pyridine (20 ml), and the resultant mixture stirred for 3.5 hours and then allowed to stand at the ambient temperature for 16 hours. The mixture was poured into water, acidified with dilute hydrochloric acid, and extracted with chloroform. After drying the extracts with anhydrous magnesium sulphate, the solvents were evaporated under reduced pressure and the residual pressure and the

residual oil identified by n.m.r. and infra red spectroscopy as 4-methoxy-2,3,5,6-tetrafluorobenzyl tetrahydropyran-2-yl ether.

(c) Preparation of 4-methoxy-2,3,5,6-tetrafluorobenzyl alcohol.

The product of step (b) above (2.8 g) was dissovled in a mixture of methyl alcohol (50 ml) and concentrated hydrochloric acid (4 ml) and the resultant mixture heated at reflux temperature for a period of 5 hours. After removal of the methanol by evaporation under reduced pressure the residual oil was partitioned between water and chloroform. The chloroform layer was separated, washed with water, dried over anhydrous magnesium sulphate, and concentrated by evaporation of the chloroform to yield 4-methoxy-2,3,5,6-tetrafluorobenzyl alcohol as a colourless oil. The identity was confirmed by n.m.r. and infra red spectroscopy.

N.m.r. ($^1H$(ppm)$CDCl_3$): 2.63 (s,1H); 4.02 (d,3H); 4.65 (d,2H).

## EXAMPLE 6

By the procedure illustrated in Example 5 4-ethylthio-2,3,5,6-tetrafluorobenzyl alcohol (b.p. 120°C/0.05 mm Hg. Kugelrohr apparatus) was obtained from pentafluorobenzyl tetrahydropyran-2-yl ether via 4-ethylthio-2,3,5,6-tetrafluorobenzyl tetrahydropyran-2-yl ether.

N.m.r. ($^1H$(ppm)$CDCl_3$): 1.24 (t,3H); 2.70 (s,1H); 2.94 (q,2H); 4.73 (s,2H).

## EXAMPLE 7

This Example illustrates the preparation of 4-ethanesulphonyl-2,3,5,6-tetrafluorobenzyl alcohol.

A mixture of 4-ethylthiotetrafluorobenzyl tetrahydropyran-2-yl ether (1.4 g), hydrogen peroxide (30% w/v, 100 vol,

4 ml) and glacial acetic acid (25 ml) was heated at the reflux temperature for 6 hours, kept at the ambient temperature for 16 hours and then heated at the reflux temperature for 4 hours. The mixture was cooled, diluted with water, and extracted with chloroform. The extracts were washed with saturated sodium bicarbonate solution, dried over anhydrous magnesium sulphate and concentrated by evaporation of the solvent under reduced pressure. The residual oil (believed to be 4-ethanesulphonyltetrafluorobenzyl tetrahydropyran-2-yl ether) was dissolved in a mixture of methanol (30 ml) and concentrated hydrochloric acid (3.0 ml) and heated at the reflux temperature for 5 hours. After removal of the volatile portion by evaporation under reduced pressure the residue was partitioned between water and chloroform, the chloroform layer washed with water, dried over anhydrous magnesium sulphate and concentrated under reduced pressure to yield 4-ethanesulphonyltetrafluorobenzyl alcohol, identified by infra red and n.m.r. spectroscopy.

N.m.r. ($^1H$(ppm)$CDCl_3$): 1.40 (t,3H); 3.21 (s,1H);
3.42 (q,2H); 4.85 (s,2H).

EXAMPLE 8

The procedure of Example 4 was used to prepare 4-allyloxy-2,3,5,6-tetrafluorobenzyl alcohol by the reaction of pentafluorobenzyl alcohol with a solution of sodium in allyl alcohol.

N.m.r. ($^1H$(ppm)$CDCl_3$): 3.20 (s,1H); 4.70 (t,4H);
5.18-5.54 (m,2H); 5.81-6.22 (m,1H).

Infra red (liquid film): 3600-3100, 2950, 1650, 1495, 1420, 1140, 1030, 980, 930 $cm^{-1}$

EXAMPLE 9

This Example illustrates the preparation of 4-(3-methyl-but-2-en-1-yl)-2,3,5,6-tetrafluorobenzyl alcohol.

(a) 2,3,5,6-tetrafluorobenzyl alcohol.

Lithium aluminium hydride (4.6 g) was added in small portions to a stirred solution of 2,3,5,6-tetrafluoro-benzoic acid (30.0 g) in dry ether (600 ml) at the ambient temperature, and stirring continued for a period of 3 hours. After decomposition of the excess lithium aluminium hydride with ethyl alcohol, water was added in excess and the ethereal phase separated, washed with water, and concentrated to yield crude 2,3,5,6-tetrafluorobenzyl alcohol (13.0 g) as a colourless oil.

N.m.r. ($^1H$(ppm)$CDCl_3$): 3.44 (s,1H); 4.72 (t,2H); 6.80-7.40 (m,1H)

Infra red (liquid film): 3600-3100, 2950, 1510, 1260, 1180, 1050, 870 $cm^{-1}$

(b) 2,3,5,6-tetrafluorobenzyl tetrahydropyranyl ether was prepared from the product of step (a) by the method set out in step (a) of Example 5.

N.m.r. ($^1H$(ppm)$CDCl_3$): 1.30-2.10 (m,6H); 3.40-4.10 (m,2H); 4.45-5.10 (m,3H); 6.86-7.32 (m,1H)

Infra red (liquid film): 2950, 1510, 1270, 1175, 1120, 1030 970, 870 $cm^{-1}$

(c) 4-(3-methylbut-2-en-1-yl)-2,3,5,6-tetrafluorobenzyl tetrahydropyranyl ether.

The product from step (b) (0.84 g) was dissolved in dry tetrahydrofuran and then stirred solution cooled to -70°C under an argon atmosphere. Lithium butyl (2.2 ml of a 1.6M solution in *n*-hexane was added slowly, after which the resultant mixture was stirred for 45 minutes at -70°C.

1-bromo-3-methylbut-2-ene (0.50 ml of a freshly distilled sample) was added slowly and after a further 30 minutes stirring at -70°C the mixture was allowed to attain the ambient temperature (ca. 25°C). Water was carefully added to the mixture which was then acidified with dilute hydrochloric acid. After keeping for 18 hours the ethereal phase was separated from the mixture, the aqueous phase extracted with diethyl ether and the extract combined with the ethereal phase. After washing with water and drying over anhydrous magnesium sulphate the ethereal solution was concentrated by evaporation of the solvents under reduced pressure and the residual oil (0.85 g) identified as a mixture of the required product with the starting material.
Infra red (liquid film): 2950, 1510, 1490, 1270, 1175, 1120, 1030, 970, 970 $cm^{-1}$

(d) 4-(3-methylbut-2-en-1-yl)-2,3,5,6-tetrafluorobenzyl alcohol was obtained (in mixture with 2,3,4,5-tetrafluorobenzyl alcohol) by subjecting the mixture prepared in step (c) to the method of step (c) of Example 5. The required product was separated from the mixture by thick layer preparative chromatography using 2 mm thick silica gel plates and ether/chloroform (1:10 by volume) as eluent.
N.m.r. ($^1H$(ppm)$CDCl_3$): 1.72 (d,6H; 2.72 (m,1H); 3.41 (m,2H); 4.80 (s,2H); 5.07-5.35 (m,1H)
Infra red (liquid film): 3600-3100, 2990, 1500, 1260, 1180 1090, 870 $cm^{-1}$

EXAMPLE 10

The procedures of Example 9 were used to prepare other 4-alkenylfluorobenzyl alcohols via their tetrahydropyranyl ethers as follows:

(i) 4-(but-3-en-1-yl)-2,3,5,6-tetrafluorobenzyl alcohol.
N.m.r. ($^1H$(ppm)$CDCl_3$): 1.48-1.78 (m,3H); 2.60 (s,1H) 3.40 (m,2H); 4.70 (s,2H); 5.25-5.75 (m,2H)
Infra red (liquid film): 3600-3100, 1495, 1275, 1030 975, 860 $cm^{-1}$

(ii) 4-(but-3-en-1-yl)-2,4,5,6-tetrafluorobenzyl tetrahydropyranyl ether (mixture with 2,3,5,6-tetrafluorobenzyl tetrahydropyranyl ether).
Infra red (liquid film): 2950, 1505, 1490, 1260, 1200, 1120, 1030, 970, 910, 870 $cm^{-1}$

(iii) 4-allyl-3,5-difluorobenzyl alcohol

N.m.r. ($^1H$(ppm)$CDCl_3$): 2.10 (m,1H); 3.40 (m,2H); 4.62 (s,2H); 4.90-5.15 (m,2H) 5.68-6.18 (m,1H); 6.85 (m,2H)
Infra red (liquid film): 3600-3100, 2950, 1640, 1585, 1435, 1315, 1215, 1190, 1115, 1030 $cm^{-1}$

(iv) 4-allyl-3,5-difluorobenzyl tetrahydropyranyl ether (mixture with 3,5-difluorobenzyl tetrahydropyranyl ether).
Infra red (liquid film): 2950, 1640, 1595, 1585, 1435, 1200, 1115, 1070, 1030, 1000, 905 $cm^{-1}$

(v) 3,5-difluorobenzyl alcohol - b.p. 200-202°C.
Infra red (liquid film): 3600-3100, 2900, 1625, 1595 1460, 1320, 1115, 955, 850 $cm^{-1}$

## EXAMPLE 11

This Example illustrates the preparation of 4-allyl-2,6-difluorobenzyl alcohol.

(a) Allyl-3,5-difluorobenzene

A solution of allyl bromide (2.5 g) in dry tetrahydrofuran (10 ml) was added dropwise with stirring to 3,5-difluorobromobenzene (4.0 g) with magnesium (0.5 g) in dry tetrahydrofuran) in dry tetrahydrofuran (40 ml), whilst maintaining the temperature of the mixture at about 20°C. When the addition was complete the mixture was allowed to warm to the ambient temperature (ca. 25°C). After a period of 18 hours the mixture was poured into water (100 ml) and the resultant mixture extracted with diethyl ether, the extract washed with water, dried over anhydrous magnesium sulphate and the ether removed by distillation at atmospheric pressure. The residual oil was purified by distillation at atmospheric pressure to yield allyl-3,5-difluorobenzene (b.p. 154-155°) as a colourless oil.

N.m.r. ($^1H$(ppm)$CDCl_3$): 3.40 (d,2H); 5.10 (dd,2H); 6.00 (m,1H); 6.70 (m,3H)

(b) 4-Allyl-3,5-difluorobenzoic acid was obtained from the product of step (a) by using the procedure of step (b) of Example 2, as a white solid, m.p. 80-82°C.

N.m.r. ($^1H$(ppm)$CDCl_3$): 3.40 (d,2H); 5.20 (dd,2H); 6.00 (m,1H); 6.90 (d,2H); 10.90 (s,1H)

Infra red (liquid paraffin): 3300-2500, 1700, 1630, 1570, 1450, 1280, 1040, 930 $cm^{-1}$

(c) 4-Allyl-3,5-difluorobenzyl alcohol was obtained by the reduction of the product of step (b) by the use of the procedure of step (c) of Example 2.

N.m.r. ($^1H$(ppm)$CDCl_3$): 3.40 (d,3H); 4.70 (s,2H); 5.20 (dd,2H); 6.00 (m,1H); 6.8 (d,2H)

EXAMPLE 12

This Example illustrates the preparation of 3-methyl-2,4,5,6-tetrafluorobenzyl alcohol.

(a) 3-bromo-2,4,5,6-tetrafluorotoluene.

A solution of dimethyl sulphate (8.0 ml) in dry tetrahydrofuran (20 ml) was added slowly to a stirred solution of 3-bromotetrafluorobenzene magnesium bromide [obtained by the reaction of magnesium (1.6 g) with 1,3-dibromotetrafluorobenzene (20 ml)] in dry tetrahydrofuran (150 ml) over 30 minutes at the ambient temperature. When the addition was complete the mixture temperature had risen to 45°C. The mixture was heated at the reflux temperature for 15 minutes and then cooled to the ambient temperature (ca. 25°C). Dilute hydrochloric acid (30 ml of a 1N solution) was added and the mixture thereafter neutralised with saturated sodium bicarbonate solution. After diluting the mixture with water (200 ml) it was extracted with ether and the extracts washed with water and dried over anhydrous magnesium sulphate. After removal of the solvent by evaporation under reduced pressure the residual oil was distilled to yield 3-bromo-2,4,5,6-tetra-fluorotoluene (9.0 g), b.p. 96-98°C/85 mm Hg.

(b) 3-methyl-2,4,5,6-tetrafluorobenzaldehyde

n-Butyl lithium (12.9 ml of a 1.6M solution in hexane) was added dropwise to a solution of 3-bromo-2,4,5,6-tetrafluorotoluene (5.0 g) in diethyl ether (40 ml) under an argon atmosphere whilst maintaining the mixture temperature within the range -60 to -70°C. When the addition was complete the mixture was stirred for a period of 1.5 hours at -70°C after which a solution of N-methylformanilide (2.8 g) in diethyl ether (15 ml) was added and the resultant mixture allowed to attain the ambient temperature (ca. 25°C). Dilute hydrochloric acid (25 ml) of a 2N solution) was added to the well-stirred mixture after which

the ethereal layer was separated, washed with water and dried over anhydrous magnesium sulphate. After removal of the solvent by evaporation under reduced pressure the residual oil was distilled to yield 3-methyl-2,4,5,6-tetrafluorobenzaldehyde (1.6 g) b.p. 78-80°C/14 mm Hg.
Infra red (liquid film): 1710, 1640, 1490, 1140 $cm^{-1}$

(c) 3-Methyl-2,4,5,6-tetrafluorobenzaldehyde.

Sodium borohydride (0.3 g) was added in small portions to a solution of 3-methyl-2,4,5,6-tetrafluorobenzaldehyde (1.5 g) in methyl alcohol (15 ml) at the ambient temperature, after which the mixture was heated at the reflux temperature for 30 minutes. The methyl alcohol was removed by evaporation under reduced pressure and the residue partitioned between dilute hydrochloric acid (20 ml of a 0.1N solution) and ether (20 ml). The ethereal phase was separated, washed with saturated sodium bicarbonate solution and with water and then dried over anhydrous magnesium sulphate. After evaporation of the ether under reduced pressure the product was distilled in a Kugelrohr apparatus to yield 3-methyl-2,4,5,6-tetrafluorobenzyl alcohol (1.2 g) - approximate b.p. 100°C/14 mm Hg.
N.m.r. ($^1H$(ppm)$CDCl_3$): 2.20 (s,3H); 3.20 (t,1H); 4.6 (m,2H)
Infra red (liquid film): 3300, 1650, 1480, 1120, 1110 $cm^{-1}$

## EXAMPLE 13

This Example illustrates the preparation of 4-amino-2,3,5,6-tetrafluorobenzyl alcohol.

Lithium aluminium hydride (0.2 g) was added in small portion to a stirred solution of 4-amino-2,3,5,6-tetrafluorobenzoic acid (1.41 g) in dry diethyl ether (20 ml) under a nitrogen atmosphere and the mixture stirred at the ambient temperature (ca. 25°C) for one hour and then heated at the reflux temperature for 3 hours. After cooling to the ambient temperature the mixture was kept for 18 hours

before water was carefully added. The mixture was acidified with dilute hydrochloric acid (2N) and extracted with ether. The extracts were washed with water dried over anhydrous magnesium sulphate and concentrated by evaporation of the ether. The resultant solid (a mixture of the desired product with the starting material) was washed with saturated sodium bicarbonate solution and the residual solid recrystallised from toluene to give 4-amino-2,3,5,6-tetrafluorobenzyl alcohol (0.3 g) m.p. 92-94°C.
N.m.r. ($^1H$(ppm)$CDCl_3$/DMSO-$d_6$): 4.60 (s,2H); 4.90 (s,3H);
Infra red (liquid paraffin): 3350, 3180, 1670, 1375, 1290, 1120, 1020, 900 $cm^{-1}$.

EXAMPLE 14

This Example illustrates the preparation of 4-methoxy-2,6-difluorobenzyl alcohol.

(a) 2,4,6-Trifluorobenzoic acid.
This was obtained from 1,3,5-trifluorobenzene by the procedure described in step (b) of Example 2. 2,4,6-Trifluorobenzoic acid was obtained as a off-white crystalline solid, m.p. 131-133°C; when recrystallised from water.
N.m.r. ($^1H(\tau)CDCl_3$): 3.26 (t,2H).
Infra red (liquid paraffin): 3750-2500, 1710 $cm^{-1}$.

(b) Methyl 2,4,6-trifluorobenzoate.
A mixture of 2,4,6-trifluorobenzoic acid (2.0 g) and thionyl chloride (10 ml) was heated at the reflux temperature for 3 hours, after which the excess thionyl chloride was removed by distillation under reduced pressure, the final traces being removed by azeotropic distillation with toluene. The residual liquid (infra red (liquid film): 1790 $cm^{-1}$ indicates 2,4,6-trifluorobenzoyl chloride, 1.5 g) was added to methanol (7.5 ml), and pyridine (2.0 ml) was added slowly to the stirred mixture. When the exothermic

reaction had subsided the mixture was kept at the ambient temperature (ca. 25°C) for 18 hours, diluted with water, acidified with dilute hydrochloric acid and extracted with diethyl ether. The ethereal extracts were washed successively with water, saturated sodium bicarbonate, and water and dried over anhydrous magnesium sulphate. After removal of the ether by evaporation the residual liquid was subjected to distillation in a Kugelrohr apparatus, to give methyl 2,4,6-trifluorobenzoate (1.1 g) having a boiling point of 105-110°C/15 mg Hg.
N.m.r. ($^1H(\tau)CDCl_3$): 3.30 (t,2H) 6.10 (s,3H).
Infra red (liquid film): 1740 $cm^{-1}$.

(c) Methyl 4-methoxy-2,6-difluorobenzoate.
A mixture of methyl 2,4,6-trifluorobenzoate (5.5 g) and a solution of sodium methoxide in methanol (obtained by dissolving sodium (0.73 g) in methanol (10 ml) was refluxed for 7 hours, after which the excess methanol was distilled off under reduced pressure. Water (50 ml) was added and the mixture extracted with diethyl ether. After drying over anhydrous magnesium sulphate the extracts were concentrated by evaporation of the ether to give a partly solidified residue. Trituration with cooled petroleum ether and filtration yielded white crystals of methyl 4-methoxy-2,6-difluorobenzoate (1.4 g) m.p. 82-84°C.
N.m.r. ($^1H(\tau)CDCl_3$): 3.54 (d,2H; 6.10 (s,3H); 6.19 (s,3H).
Infra red (liquid paraffin): 1735 $cm^{-1}$.

(d) 4-Methoxy-2,6-difluorobenzyl alcohol.
A solution of 'Red-Al' (1.5 g of a 70% solution of $NaAlH_2(OCH_2CH_2OCH_3)_2$ in toluene) was added slowly to a stirred solution of methyl 4-methoxy-2,6-difluorobenzoate (1.0 g) in dry tetrahydrofuran (10 ml) at the ambient temperature. After the exothermic reaction had subsided the mixture was stirred for 30 minutes and ethyl acetate (10 ml) and water (200 ml) acidified with dilute

hydrochloric acid (5 ml) were added. After extracting the mixture with ether (2 x 30 ml) the combined extracts were dried over anhydrous magnesium sulphate and concentrated by evaporation of the ether. The residual oil was purified by flash chromatography on silica using an ethyl acetate/-petroleum ether eluent to give 4-methoxy-2,6-difluorobenzoyl alcohol (0.62 g).

N.m.r. ($^1H(\tau)CDCl_3$): 3.59 (d,2H); 5.34 (s,2H); 6.20 (s,3H)

Infra red (liquid film): 3800 $cm^{-1}$

EXAMPLE 15

This Example illustrates the preparation of 2,3,5,6-tetrafluorotoluene.

A solution of n-butyllithium in hexane (1.6M, 62.5 ml) was added dropwise to a well stirred solution of 1,2,4,5-tetrafluorobenzene (15.0g) in dry tetrahydrofuran (150 ml) maintained at a temperature of -60°C under an atmosphere of dry argon. When the addition was complete the mixture was stirred at -45°C for 2 hours and then methyl iodide (14.2g) was added dropwise whilst the temperature was kept at -45°C. After a period of 30 minutes the mixture was allowed to warm to the ambient temperature, poured into distilled water and the mixture extracted with diethyl ether (2 x 50 ml), and the extracts dried over anhydrous magnesium sulphate. After filtering the solution was concentrated by evaporation of the solvents at atmospheric pressure. The residual oil was distilled and the fraction boiling in the range 117-121°C at atmospheric pressure 6.0 g) collected, identified by n.m.r. and gas chromatographic analysis as consisting of ca. 95% of the required 2,3,5,6-tetrafluorotoluene and ca. 5% of 2,3,5,6- tetra-fluoro-1,4-xylene.

N.m.r. ($^1H(ppm)CDCl_3$): 2.28(t,3H); 6.58-6.94 (m,1H). Infra red (liquid film): 3075, 1645, 1510, 1255, 1165 $cm^{-1}$.

EXAMPLE 16

This Example illustrates the preparation of 2,3,5,6-tetrafluoro-4-toluic acid.

The product of Example 15 above (5.5g) was mixed with diethyl ether (35 ml), the mixture cooled to -70°C, and maintained at this temperature whilst a solution of n-butyllithium in h-hexane (1.6M, 21 ml) was slowly added. The mixture was stirred for a period of 1 hour during which time a fine white precipitate was formed. Dry carbon dioxide gas was then passed into the mixture for 30 minutes whilst the temperature was maintained within the range -70°C to -40°C, and continued to be passed in thereafter whilst the mixture was allowed to warm to the ambient temperature.

After acidifying with dilute hydrochloric acid (6N, 40 ml) the organic phase was separated, washed with water and dried over anhydrous magnesium sulphate. After evaporation of the solvents under reduced pressure the residual oil (which from n.m.r. analysis was shown to be an approximately 1:1 mixture of the desired product and pentanoic acid) was carefully distilled under reduced pressure (water pump) using a Kugelrohr apparatus, and the fraction which solidified on cooling collected and recrystallised from toluene to yield 2,3,5,6-tetrafluoro-4-toluic acid, m.p. 170°C (0.65g), identified by infra red and nuclear magnetic resonance spectroscopy.

N.m.r. ($^1H$(ppm)$CDCl_3$): 2.44(t,3H); 11.56 (s,1H) Infra red (liquid paraffin): 3300-2450, 1710, 1650, 1460, 1070 $cm^{-1}$.

EXAMPLE 17

This example illustrates the preparation of 2,3,5,6-tetrafluorobenzyl bromide.

0060617

A mixture of 2,3,5,6-tetrafluorotoluene (1.7g), N-bromosuccinimide (1.9g), dry carbon tetrachloride (10 ml) and benzoyl peroxide (0.01g) was heated at the reflux temperature for 20 hours, cooled to the ambient temperature (ca.25°C) filtered and the filtrate diluted with diethyl ether. The ethereal solution was washed with water, dried over anhydrous magnesium sulphate and concentrated by evaporation of the solvents to yield 2,3,5,6-tetrafluorobenzyl bromide as a mobile colourless oil.

EXAMPLE 18

This Example illustrates the preparation of methyl 4-methyl 2,3,5,6-tetrafluorobenzoate.

A mixture of 2,3,5,6-tetrafluoro-4-toluic acid (1.0g), methyl alcohol (5 ml) and concentrated sulphuric acid (0.25 ml) was heated at the reflux temperature for 10 hours, cooled to the ambient temperature (ca. 25°C) and poured into iced water. The resultant mixture was extracted with diethyl ether, the extracts washed with water, dried over anhydrous magnesium sulphate and concentrated by evaporation of the ether under reduced pressure. The residual oil was shown by infra red analysis (liquid film : 1740 $cm^{-1}$) and N.m.r. spectroscopy to be the required methyl 4-methyl 2,3,5,6-tetrafluorobenzoate.

EXAMPLE 19

This Example illustrates the preparation of N,N-diethyl-2,3,5,6-tetrafluorobenzylamine.

A solution of diethylamine (0.6g) in dry diethyl ether (2.0 ml) was added slowly to a stirred solution of 2,3,5,6-tetrafluorobenzyl bromide (2.0g) in diethyl ether (30 ml) at the ambient temperature (ca. 25°C), and the resultant mixture stirred for a further 6 hours and then kept at the ambient temperature for a further 18 hours. After removal of the ether by evaporation water containing a few drops of dilute hydrochloric acid was added to the residue and the mixture obtained washed with ether, made basic with saturated sodium bicarbonate solution and extracted with ether. The ethereal extracts were washed with water, dried over anhydrous magnesium sulphate and concentrated by evaporation of the ether. The residual oil was shown by infra red and N.m.r. spectroscopy to be N,N-diethyl-2,3,5,6-tetrafluorobenzylamine.

EXAMPLE 20

The procedure of Example 16 was used to convert diethylaminomethyl-2,3,5,6-tetrafluorobenzene to 4-diethylaminomethyl-2,3,5,6-tetrafluorobenzoic acid.

EXAMPLE 21

The procedure of Example 18 was used to convert diethylaminomethyl-2,3,5,6-tetrafluorobenzoic acid to its methyl ester.

EXAMPLE 22

This Example illustrates the preparation of 4-diethylaminomethyl-2,3,5,6-tetrafluorobenzyl alcohol.

Lithium aluminium hydride (50 mg) was added carefully to a stirred solution of methyl 4-diethylamino-2,3,5,6-tetrafluorobenzoate (0.79g) in dry diethyl ether (10 ml) at the ambient temperature. After 1 hour the mixture was heated at the reflux temperature for 7 hours after which the mixture was cooled and partitioned between more ether and water. The ethereal phase was separated and combined with a further ethereal extract of the aqueous phase. The combined extracts were washed with water, dried over anhydrous magnesium sulphate, and the ether removed by evaporation under reduced pressure. The residual oil was purified by preparative thick layer chromatography using 2 mm thick silica gel and a 1:1 ether/petroleum ether mixture as eluent. The required product as obtained (after removal from the plate of the component of largest Rf value) by extraction of the silica with chloroform methyl alcohol mixture and identified by N.m.r. and infra red spectroscopy.

N.m.r. ($CDCl_3$) : 1.10 (t,6H); 2.54 (q,4H); 3.75 (m,2H); 4.04 (S,1H); 4.72 (m,2H) ppm.

Infra red (liquid fim) : 3600-3100, 2980, 1490, 1280, 1050, 880 $cm^{-1}$.

EXAMPLE 23

This Example illustrates the preparation of ethyl 2,3,5,6-tetrafluorobenzyl ether.

2,3,5,6-tetrafluorobenzyl bromide (2.43 g) was added to a stirred solution of sodium (0.27 g) in ethyl alcohol (20 ml) at the ambient temperature, and the mixture stirred for a further 90 minutes. After keeping at the ambient temperature for 18 hours the mixture was poured into an excess of water and the resultant mixture extracted three

times with diethyl ether. The combined extracts were washed with water, dried over anhydrous magnesium sulphate and concentrated by evaporation of the volatile portion under reduced pressure. The residual oil (1.6 g) was shown N.m.r. and infra red spectroscopy to be the required product.

EXAMPLE 24

The procedure of Example 23 was used to prepare other compounds from α-bromo-2,3,5,6-tetrafluorotoluene, and the appropriate hydroxy compound as follows :-

(i) *n*-propoxymethyl-2,3,5,6-tetrafluorobenzene

(ii) phenoxymethyl-2,3,5,6-tetrafluorobenzene.

EXAMPLE 25

The procedure of Example 16 was used to prepare the following benzoic acids from the appropriate precursors as follows :-

(i) 4-ethoxymethyl-2,3,5,6-tetrafluorobenzoic acid

(ii) 4-*n*-propoxymethyl-2,3,5,6-tetrafluorobenzoic acid

(iii) 4-phenoxymethyl-2,3,5,6-tetrafluorobenzoic acid.

EXAMPLE 26

The procedure of Example 22 was used to reduce the appropriate benzoic acids to the following benzyl alcohols :-

(i) 4-ethoxymethyl-2,3,5,6-tetrafluorobenzyl alcohol.

N.m.r. ($CDCl_3$) : 1.20 (t,3H); 2.82 (s,1H); 3.58 (q,2H); 4.61 (m,2H); 4.76 (m,2H) ppm

Infra red (liquid film) : 3600-3100, 2980, 1490, 1290, 1060, 880 $cm^{-1}$

(ii) 4-n-propoxymethyl-2,3,5,6-tetrafluorobenzyl alcohol.

N.m.r. ($CDCl_3$) : 0.92 (t,3H); 1.60 (q,2H); 3.46 (m,3H); 4.60 (m,2H); 4.77 (m,2H) ppm

Infra red (liquid film) : 3600-3100, 2980, 1490, 1290, 1060, 880 $cm^{-1}$

(iii) 4-phenoxymethyl-2,3,5,6-tetrafluorobenzyl alcohol.

N.m.r. ($CDCl_3$) : 2.10 (s,1H); 4.60 (m,2H); 5.10 (m,2H); 6.85-7.38 (m,5H) ppm

Infra red (liquid film) : 3600-3100, 2590, 1600, 1490, 1290, 1060, 1040, 890 $cm^{-1}$

## EXAMPLE 27

The procedure of Example 17 was used to convert the methyl ester of 2,3,5,6-tetrafluoro-4-toluic acid into methyl 4-bromomethyl-2,3,5,6-tetrafluorobenzoate.

## EXAMPLE 28

The procedure of Example 23 was used to prepare the following compounds from methyl 4-bromomethyl-2,3,5,6-tetrafluorobenzoate and the appropriate alcohol or thiol.

(i) methyl 4-methoxymethyl-2,3,5,6-tetrafluorobenzoate.

(ii) methyl 4-ethylthiomethyl-2,3,5,6-tetrafluorobenzoate.

(iii) methyl 4-methylthiomethyl-2,3,5,6-tetrafluorobenzoate.

EXAMPLE 29

The procedure of Example 22 was used to obtain the following benzyl alcohols by reduction of the appropriate methyl esters as follows :-

(i) 4-methoxymethyl-2,3,5,6-tetrafluorobenzyl alcohol.

N.m.r. ($CDCl_3$) : 2.82 (s,1H); 3.44 (s,3H); 4.64 (s,2H);4.86 (s,2H) ppm

Infra red )liquid film) : 3650-3000, 1650, 1490, 1280, 1080, 1050 $cm^{-1}$

(ii) 4-ethylthiomethyl-2,3,5,6-tetrafluorobenzyl alcohol.

N.m.r. ($CDCl_3$) : 1.26 (t,3H); 2.00 (s,1H); 2.54 (q,2H); 3.78 (s,2H); 4.78 (s,2H) ppm

Infra red (liquid film) : 3650-3000, 1655, 1490, 1010, 945 $cm^{-1}$

(iii) 4-methylthiomethyl-2,3,5,6-tetrafluorobenzyl alcohol.

EXAMPLE 30

This Example illustrates the preparation of 4-allyl-2,3,5,6-tetrafluorobenzyl 2,2,3,3-tetramethylcyclopropane carboxylate. (Compound no.3, Table I).

(a) 1-Chlorocarbonyl-2,2,3,3-tetramethylcyclopropane.

2,2,3,3-Tetramethylcyclopropane carboxylic acid (8.25 g) was added (in approximately 1 gm aliquots) to hot thionyl chloride (50 ml) maintained at a temperature just less than the reflux temperature. When the addition was completed the mixture was heated at the reflux temperature for 4 hours. After cooling to the ambient temperature (ca. 25°C) the excess thionyl chloride was removed from the mixture by evaporation under reduced pressure at a temperature not exceeding 35°C, to give 1-chlorocarbonyl-2,2,3,3-tetramethylcyclopropane (9.2 g) as a brown oil which partially crystallised as standing.

N.m.r. ($CDCl_3$) $\tau$: 8.14 (s,1H); 8.70 (s,12H).

Infra red (liquid film): 1780 $cm^{-1}$

(b) 4-Allyl-2,3,5,6-tetrafluorobenzyl 2,2,3,3-tetramethylcyclopropane carboxylate

A mixture of 1-chlorocarbonyl-2,2,3,3-tetramethylcyclopropane (0.80 g), 4-allyl-2,3,5,6-tetrafluorobenzyl alcohol (1.1 g), pyridine (0.40 g) and methylene chloride (10 ml) was stirred at the ambient temperature (ca. 25°C) for 5 hours. The mixture was then washed with dilute hydrochloric acid, dilute sodium bicarbonate solution and with water, and dried over anhydrous magnesium sulphate. After removal of the solvent by evaporation under reduced pressure the residual oil was purified by preparative TLC using a 19:1 mixture by volume of petroleum ether (boiling range 40-60°C) and diethyl ether as eluent) to give 4-allyl-2,3,5,6-tetrafluorobenzyl 2,2,3,3-tetramethylcyclopropane carboxylate (1.24 g) as a pale yellow oil.

N.m.r. ($CDCl_3$) $\tau$: 3.80-4.30 (m,1H); 4.80 (s,2H); 4.80-5.00 (m,2H); 5.50 (d,2H); 8.75 (s,7H); 8.82 (s,6H)

Infra red (liquid film): 1720 $cm^{-1}$

## EXAMPLE 31

By the use of a procedure similar to that illustrated in the previous Example the following compounds were made by reacting 1-chlorocarbonyl-2,2,3,3-tetramethylcyclopropane with the appropriate alcohols as stated.

(i) Compound no. 1 (Table I) from 2,3,4,5,6-pentafluorobenzyl alcohol.
N.m.r.($CDCl_3$) $\tau$: 4.78 (s,2H); 8.74 (s,7H); 8.80 (s,6H)
Infra red (liquid film): 1720 $cm^{-1}$

(ii) Compound no.2 (Table I), m.p. 50-51°C, from 4-methyl-2,3,5,6-tetrafluorobenzyl alcohol.
N.m.r.($CDCl_3$) $\tau$: 4.84 (s,2H); 7.74 (s,3H); 8.74 (s,7H) 8.80 (s,6H)
Infra red (liquid paraffin): 1720 $cm^{-1}$

(iii) Compound no.4 (Table I) from 4-methoxy-2,3,5,6-tetrafluorobenzyl alcohol.
N.m.r.($CDCl_3$) $\tau$: 4.84 (s,2H); 5.86 (s,3H); 8.74 (s,7H) 8.80 (s,6H)
Infra red (liquid film): 1720 $cm^{-1}$

(iv) Compound no.5 (Table I) from 4-methoxymethyl-2,3,5,6-tetrafluorobenzyl alcohol.
N.m.r.($CDCl_3$) $\tau$: 4.80 (s,2H); 5.40 (s,2H); 6.60 (s,3H) 8.76 (s,7H); 8.82 (s,6H)
Infra red (liquid film): 1720 $cm^{-1}$

(v) Compound no.6 (Table I) m.p. 49-50°C, from 2-chloro-6-fluorobenzyl alcohol.
N.m.r.($CDCl_3$) $\tau$: 2.60-3.10 (m,3H); 4.70 (s,2H); 8.70 (s,7H); 8.81 (s,6H).
Infra red (liquid film): 1720 $cm^{-1}$

(vi) Compound no.7 (Table I) from 2-chloro-4-fluorobenzyl alcohol.
N.m.r.($CDCl_3$)$\tau$: 2.50 (q,1H); 2.60-3.10 (m,2H); 4.80 (s,2H); 8.70 (s,7H); 8.76 (s,6H)

(vii) Compound no.19 (Table I) from 4-benzyl-2,3,5,6-tetrafluorobenzyl alcohol (m.p. 109-110°C).
N.m.r.($CDCl_3$) $\delta$ : 1.20 (s,6H); 1.28 (s.6H); 4.12 (s,2H); 5.23 (s,2H); 7.36 (s,5H)
Infra red (liquid paraffin) : 1712 $cm^{-1}$

(viii) Compound no.45 (Table I) from 4-methylthiomethyl-2,3,5,6-tetrafluorobenzyl alcohol.
N.m.r. ($CDCl_3$) $\delta$ : 1.20 (s,6H); 1.28 (s,6H); 2.16 (s,3H); 3.82 (s,2H); 5.24 (s,2H)
Infra red (liquid paraffin) : 1720 $cm^{-1}$

(ix) Compound no.37 (Table I) from 4-diethylaminoethyl-2,3,5,6-tetrafluorobenzyl alcohol (m.p. 49-52°C)
N.m.r. ($CDCl_3$) $\delta$ : 1.10 (t,6H); 1.20 (s,6H); 1.21 (s.6H); 2.58 (q,4H); 3.80 (s.2H); 5.22 (s,2H)
Infra red (liquid film) : 1712 $cm^{-1}$

NDB/aji/SPEC216
Jan 25 1982

1. A compound of formula :-

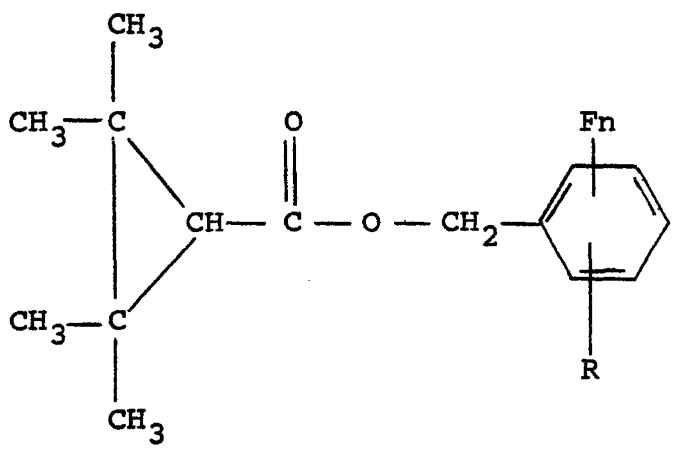

wherein n represents an integer from one to four, and R represents hydrogen, halo, alkyl of up to four carbon atoms, alkenyl of up to five carbon atoms, benzyl, alkoxy of up to four carbon atoms, alkenyloxy of up to five carbon atoms, alkylthio of up to four carbon atoms, alkanesulphonyl of up to four carbon atoms, amino, acylamino of up to four carbon atoms, alkylamino of up to four carbon atoms, dialkylamino of up to a total of eight carbon atoms, N-(alkyl)acylamino of up to a total of eight carbon atoms, alkoxyalkyl of up to a total of six carbon atoms, aryloxyalkyl of up to a total of nine carbon atoms, alkenyloxyalkyl of up to a total of six carbon atoms, alkylthioalkyl of up to a total of six carbon atoms, alkylaminoalkyl of up to a total of six carbon atoms or dialkylaminoalkyl of up to a total of ten carbon atoms.

2. A compound according to claim 1 wherein n is four.

3. A compound according to claim 2 wherein R is selected from hydrogen, fluoro, chloro, bromo, methyl, ethyl, propyl, butyl, allyl, butenyl, methylbutenyl, benzyl, methoxy, ethoxy, allyloxy, ethylthio, ethanesulphonyl, amino, acetamido, diethylamino, N-methylacetamido, methoxymethyl, ethoxymethyl, propoxymethyl, phenoxymethyl, allyloxymethyl,

methylthiomethyl, ethylthiomethyl and diethylaminomethyl.

4. A compound according to claim 3 wherein R is selected from 4-fluoro, 4-methyl, 4-methoxy, 4-allyl and 4-methoxymethyl.

5. A compound according to claim 1 wherein n is one and R represents halo.

6. A compound according to claim 5 wherein R is 2-chloro.

7. 2-Chloro-6-fluorobenzyl 2,2,3,3-tetramethyl cyclopropane carboxylate.

8. An insecticidal composition comprising an insecticidally effective amount of a compound as defined in claim 1 in association with an inert diluent or carrier material.

9. A method of combatting insect pests at a locus which comprises treating the locus with an insecticidally effective amount of the composition of claim 8.

10. A process for preparing a compound as defined in claim 1 wherein the acid halide of tetramethyl cyclopropane carboxylic acid is reacted with an alcohol of formula :-

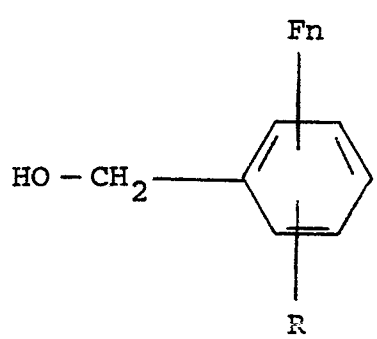

where n and R have any of the meanings given in claim 1, in the presence of a hydrogen halide acceptor at a temperature within the range 0 to 100°C.

European Patent Office

# EUROPEAN SEARCH REPORT

0060617

Application number

EP 82300641.6

## DOCUMENTS CONSIDERED TO BE RELEVANT

| Category | Citation of document with indication, where appropriate, of relevant passages | Relevant to claim |
|---|---|---|
| P,X | EP - A1 - 0 029 515 (BAYER AG) (03-06-1981)<br>* Examples 1-3; claims *<br>& IL-A-61 418 (31-12-1980)<br>-- | 1-3,5, 6,8-10 |
| A | GB - A - 1 178 857 (SUMITOMO)<br>* Example P2; pages 17-26 *<br>---- | 1,8-10 |

**CLASSIFICATION OF THE APPLICATION (Int. Cl. 3)**

C 07 C 69/74
C 07 C 147/06
C 07 C 149/36
C 07 C 103/38
C 07 C 93/26
C 07 C 149/18
C 07 C 93/227
A 01 N 53/00
C 07 C 67/14

**TECHNICAL FIELDS SEARCHED (Int.Cl. 3)**

C 07 C 67/00
C 07 C 69/00
C 07 C 147/00
C 07 C 149/00

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons
&: member of the same patent family, corresponding document

X The present search report has been drawn up for all claims

| Place of search | Date of completion of the search | Examiner |
|---|---|---|
| VIENNA | 17-06-1982 | HOFBAUER |

EPO Form 1503.1 06.78